# EUROPEAN PATENT APPLICATION

(11) **EP 1 252 907 A1**
(43) Date of publication of application: **30.10.2002**
(21) Application number: 02076634.1
(22) Date of filing: 23.04.2002
(51) Int. Cl.: A61M 5/32, A61M 5/50

(54) **Protective device for a fillable injection syringe**

(30) Priority: 25.04.2001 US 843282
(71) Applicant: Asbaghi, Hooman A., Del Mar, California 92014 (US)
(72) Inventor: Asbaghi, Hooman A., Del Mar, California 92014 (US)
(74) Representative: Shortt, Peter Bernard

(57) **Abstract**

A device (12) for covering and protecting the hollow needle (14) of a syringe (10) after the syringe (10) is used to inject a medicament into a patient (54) includes a cylindrical adapter (20) formed with a coupling (26). A syringe body (34) formed with a medicament chamber (36) is attached to the coupling (26). The hollow needle (14) is also attached to the coupling (26) in fluid communication with the medicament chamber (36). The adapter (20) is formed with a plug (46) that extends from the adapter (20). The adapter (20) is disposed within a cylindrical barrel piece (18) to allow axial movement between the adapter (20) and barrel piece (18). A slot (48) is formed in the barrel piece (18) for engagement with the adapter plug (46). The specially designed slot (48) regulates the distance the needle tip (30) extends from the nose (42) of the barrel piece (18) during a sequence of steps that include filling the syringe (10) with fluid medicament from a medicament vial (52) and then injecting a patient (54) with the medicament.

## Description

This application is a continuation-in-part of Application Serial No. 09/336,405 filed June 18, 1999, which is currently pending. The contents of Application Serial No. 09/336,405 are incorporated herein by reference.

### FIELD OF THE INVENTION

The present invention pertains generally to syringes for medical use. More particularly, the present invention pertains to protective devices for injection syringes that are filled by the user prior to the administration of an injection. The present invention is particularly, but not exclusively, useful for passively covering and protecting the needle of an injection syringe after its use.

### BACKGROUND OF THE INVENTION

Recent research from the Centers for Disease Control and Prevention (CDC) shows that approximately 384,000 needle sticks or similar injuries occur among health care workers in U.S. hospitals each year. Unfortunately, each accidental needle stick has the potential to expose a health care worker to a life-threatening virus such as hepatitis or HIV. In addition to the needle sticks that occur in hospitals, accidental needle sticks can also occur in other health care settings. For example, needle stick injuries can occur at clinics or during home health-care. In fact, some studies have estimated that over 600,000 needle sticks occur in the U.S. each year, and approximately 1,000 of these accidental needle sticks result in a life-threatening infection.

For each accidental needle stick, health care providers are obligated to test and counsel the exposed worker. Further, follow-up testing for HIV must be conducted approximately six months after the exposure. It is to be appreciated that the costs associated with the testing, lab work, the workers lost time, and the associated tracking and administrative costs, can be considerable.

Accidental needle sticks can occur in several ways. For example, sudden movement by the patient can cause a health care worker to lose control of a syringe, resulting in injury. Attempts to manually recap a needle following an injection can also result in injury. Moreover, injuries often result when contaminated unprotected needles are left unattended or disposed of improperly. In addition to accidental needle sticks, unnecessary exposure to bloodborne pathogens can result when a health care worker mistakenly reuses a contaminated needle on a patient.

In light of the above, it is an object of the present invention to provide a protective device that passively covers and protects the needle of a medical syringe after first filling the syringe with medicament and then injecting the medicament into a patient. It is another object of the present invention to provide a protective device for the needle of a medical syringe that regulates the amount of needle that is exposed during a sequence of steps that include filling the syringe with fluid medicament from a medicament vial and then injecting a patient with the medicament. It is yet another object of the present invention to provide a protective device that prevents re-use of a contaminated syringe by providing a clear, visible indication that the syringe has been used. Yet another object of the present invention is to provide a protective device for a medical syringe that is easy to use, relatively simple to implement, and comparatively cost effective.

### SUMMARY OF THE PREFERRED EMBODIMENTS

A device for protecting a hollow needle, after it has been used with a syringe to inject a fluid medicament into a patient, includes a cylindrical-shaped barrel piece that is slidably mounted over the needle to completely cover or selectively expose different lengths of the hollow needle. Specifically, a relatively short portion of the needle is exposed prior to filling the syringe with the medicament, and subsequently prior to injecting the medicament into the patient. After an injection, however, the barrel piece completely covers the hollow needle.

In accordance with the present invention, the device includes an adapter that is attached to the proximal end of the needle. With this attachment, the needle extends distally from the adapter to define an axis. The barrel piece, which is formed with an aperture, is then slidably mounted on the adapter with the needle projecting through the aperture to allow for axial movement of the barrel piece over the needle. Additionally, a biasing mechanism, such as a spring, is mounted between the adapter and the barrel piece to urge the barrel piece in a distal direction, relative to the adapter.

An important aspect of the present invention involves the cooperative interaction between the adapter and the barrel piece. To effect this interaction, a plug extends from the adapter and into a slot that is formed on the barrel piece. In detail, this slot is formed with a start position (first position), and two axially aligned, parallel tracks which sequentially establish four additional positions (second, third, fourth and fifth positions). Further, one of these tracks (a first track) is positioned between the start position and the other track (a second track). Within this configuration, relative to the axis, the start position is azimuthally offset from the first track, and is on the opposite side of the first track from the second track. Further, a first guide ramp is formed in the slot to direct the plug in its movement from the start position into the first track. Also, a second guide ramp is formed in the slot to direct the plug in its movement from the first track into the second track.

In operation, the plug on the adapter is initially located in the start position with a relatively short portion of the hollow needle extending distally from the barrel piece. In this configuration, the adapter is engaged with the syringe to establish fluid communication between the syringe and the hollow needle. To then fill the syringe with fluid medicament, the needle is inserted through the stopple of a medicine vial. In response, the medicine vial pushes against the barrel piece to move it in a proximal direction back over the adapter. This also moves the plug from the start position. Specifically, the plug is directed by the first guide ramp into the first track. Further proximal movement of the barrel piece continues until the plug is stopped in the second position at the distal end of the first track. When the needle is disengaged from the medicine vial, the biasing mechanism urges the barrel piece forward in a distal direction until the plug is stopped at the proximal end of the first track (third position). In this configuration, a relatively short portion of the needle again extends distally from the barrel piece. The syringe is now ready for injecting the fluid medicament into the patient.

With the syringe filled and the distal end of the hollow needle slightly exposed, the needle can be accurately positioned against the patient for an injection. Then, as the needle is pushed into the body of the patient, a reaction force is generated against the barrel piece. This reaction force causes the barrel piece to again move in a proximal direction back over the adapter. This time, however, the plug is directed by the second guide ramp into the second track. This movement continues until the plug is stopped in a fourth position at the distal end of the second track. The syringe can then be used to inject the fluid medicament into the patient.

Upon the completion of an injection, the needle is withdrawn from the patient and the reaction force of the body against the barrel piece is removed. The biasing mechanism then causes the plug to move out of the fourth position and along the second track and into the fifth position at the proximal end of the second track. For the present invention, this fifth position can be azimuthally offset from the second track, and it can be configured with a detent that will prevent further axial movement of the plug relative to the slot. Stated differently, the detent (fifth position) will effectively lock the barrel piece to the adapter and over the hollow needle. Importantly, in this configuration, the barrel piece completely covers the hollow needle to protect the user from unwanted needle sticks after the injection has been completed.

The invention also relates to a method for passively protecting a used syringe after injecting a medicament into a patient, said method comprising the steps of:
providing a syringe having a hollow needle, an adapter formed with plug, a barrel piece formed with a slot, and a medicament chamber, said hollow needle and said medicament chamber attached to said adapter with said needle in fluid communication with said chamber, said barrel piece having a distal end formed with an aperture for receiving said needle therethrough, said barrel piece disposed on said adapter for relative motion thereto with said plug inserted in said slot to restrict relative movement between said barrel piece and said adapter, said syringe having a spring positioned between said adapter and said barrel piece to urge said barrel piece and said adapter in opposite axial directions;
inserting said hollow needle into a vial to establish contact between said barrel piece and the vial;
continuing said inserting step to urge said barrel piece against said vial, move said plug along said slot, and further extend said needle from said barrel piece;
drawing medicament from said vial and into said syringe;
withdrawing said needle from said vial to have said biasing means move said plug along said slot and retract said barrel piece over a portion of said needle;
establishing contact between said barrel piece and the patient on pressing said hollow needle into the patient;
continuing said pressing step to urge said barrel piece against the patient, move said plug along said slot, and further extend said needle from said barrel piece; and
after injection removing said needle from the patient to have said biasing means relocate said plug along said slot and cover and protect said needle tip with said barrel piece.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Fig. 1 is a prospective view of a syringe having a protective device in accordance with the present invention;
Fig. 2 is a sectional view of a portion of the syringe as seen along line 2-2 in Fig. 1;
Fig. 3 is a plan view of an adapter in accordance with the present invention;
Fig. 4A is a plan view of a portion of a syringe, shown with the protective device in its initial position wherein the needle tip is slightly extended for locating the opening of a medicament vial;
Fig. 4B is a plan view of a portion of a syringe, shown with the protective device in its second position wherein the needle tip is extended for insertion into a medicament vial to allow the syringe to be filled with medicament;
Fig. 4C is a plan view of a portion of a syringe, shown with the protective device in its third position wherein the needle tip is slightly extended for locating an injection point on a patient;
Fig. 4D is a plan view of a portion of a syringe, shown with the protective device in its fourth position wherein the needle tip is extended to the proper penetration depth for an injection; and
Fig. 4E is a plan view of a portion of a syringe, shown with the protective device in its final position wherein the needle tip is covered and protected by the barrel piece.

### DESCRIPTION OF THE PREFERRED EMBODIMENT

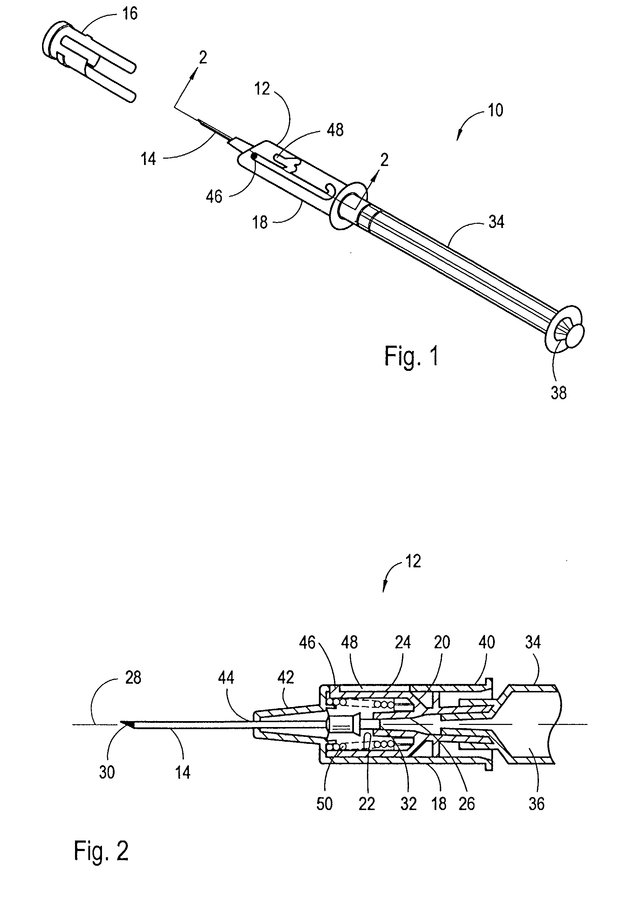

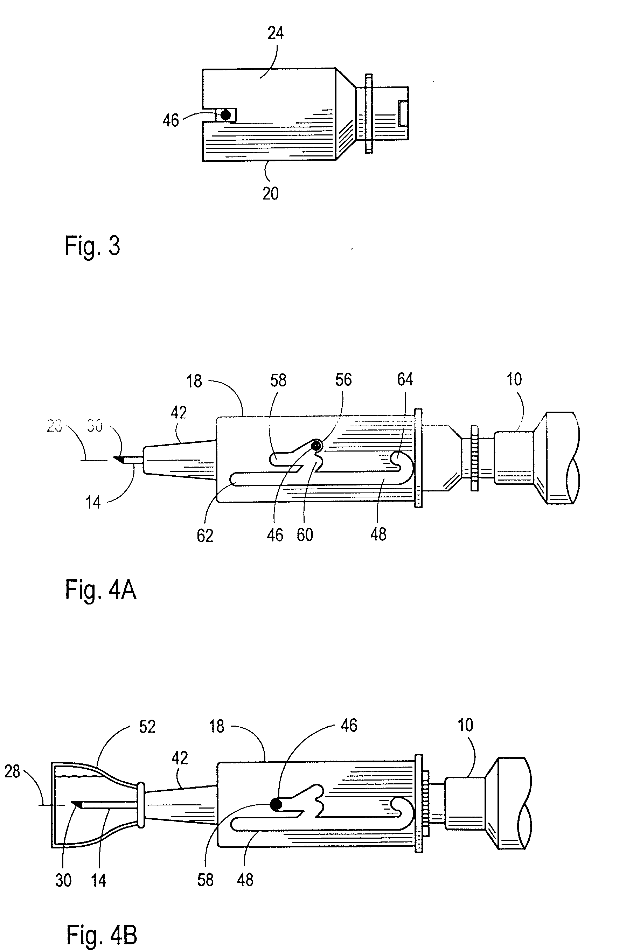

Referring to Fig. 1, a syringe in accordance with the present invention is shown and generally designated 10. As shown in Fig. 1, the syringe 10 includes a protective device 12 for covering the hollow needle 14 of the syringe 10 after use. An optional cap 16 can be included as part of the syringe 10 to cover the exposed part of the needle 14 before use. Referring now to Fig. 2, it can be seen that the protective device 12 includes a barrel piece 18 and an adapter 20. With cross reference to Figs. 2 and 3, it can be appreciated that, the adapter 20 is preferably shaped as a hollow cylinder having an inner wall 22 and an outer wall 24.

In accordance with the present invention, the adapter 20 is formed with a coupling 26, such as a luer type coupling, mounted on the inner wall 22 of the adapter 20. As shown, the coupling 26 attaches the hollow needle 14 to the adapter 20. It is to be appreciated that preferably a substantially straight, hollow needle 14 (thereby defining axis 28) is used in conjunction with the present invention. The distal end 30 of the hollow needle 14 is formed as a sharp tip suitable for piercing the skin of the patient, while the proximal end 32 is attached to the adapter 20 via the coupling 26. For the present invention, the cylindrical adapter 20 is centered on the needle axis 28 when the needle 14 is attached to the adapter 20, as shown in Fig. 2.

Referring now to Figs. 1 and 2, it can be seen that the coupling 26 also attaches the adapter 20 to the body 34 of the syringe 10. As shown, the body 34 is formed with a medicament chamber 36. Further shown, the coupling 26 attaches the body 34 of the syringe 10 to the adapter 20 with the medicament chamber 36 in fluid communication with the hollow needle 14. The syringe 10 also includes a plunger 38, engageable with the medicament chamber 36. With this cooperation of structure, the plunger 38 can be advanced into the medicament chamber 36 to expel fluid from the chamber 36, through the hollow needle 14, and then out of the distal end 30 of the needle 14.

Referring still with cross reference to Figs. 1 and 2, it can be seen that the barrel piece 18 is formed with a cylindrically shaped wall having an inner radius large enough to allow the adapter 20 to be disposed within the barrel piece 18. As shown, an optional nose 42 is formed in the barrel piece 18, extending from the cylindrical wall 40 at one end. The other end of the cylindrical barrel piece 18 is preferably open. As shown, the nose 42 of the barrel piece 18 is preferably closed except for a small aperture 44 for receiving the hollow needle 14.

Referring now with cross reference to Figs. 1, 2 and 3, it can be seen that the adapter 20 is formed with a plug 46 that extends radially from the outer wall 24 of the adapter 20. Further shown, a multi-branch slot 48 extends through the cylindrical wall 40 of the barrel piece 18 for engagement with the plug 46 of the adapter 20. With this combination of structure, the barrel piece 18 can be positioned over the adapter 20 with the plug 46 formed in the adapter 20 extending into the slot 48 formed in the barrel piece 18, as shown in Fig. 2. Further, as shown, the barrel piece 18 is substantially centered on the axis 28 of the needle 14 when positioned on the adapter 20. Fig. 2 further shows that a spring 50 is compressed inside the barrel piece 18 with one end of the spring 50 in contact with the adapter 20 and one end of the spring 50 in contact with the barrel piece 18. With the spring 50 positioned in this manner, the barrel piece 18 and adapter 20 are urged in opposite directions along the needle axis 28. It is to be appreciated that the barrel piece 18 and adapter 20 are capable of both axial and rotational movement relative to each other, restricted only by the limited ability of the plug 46 to move within the slot 48.

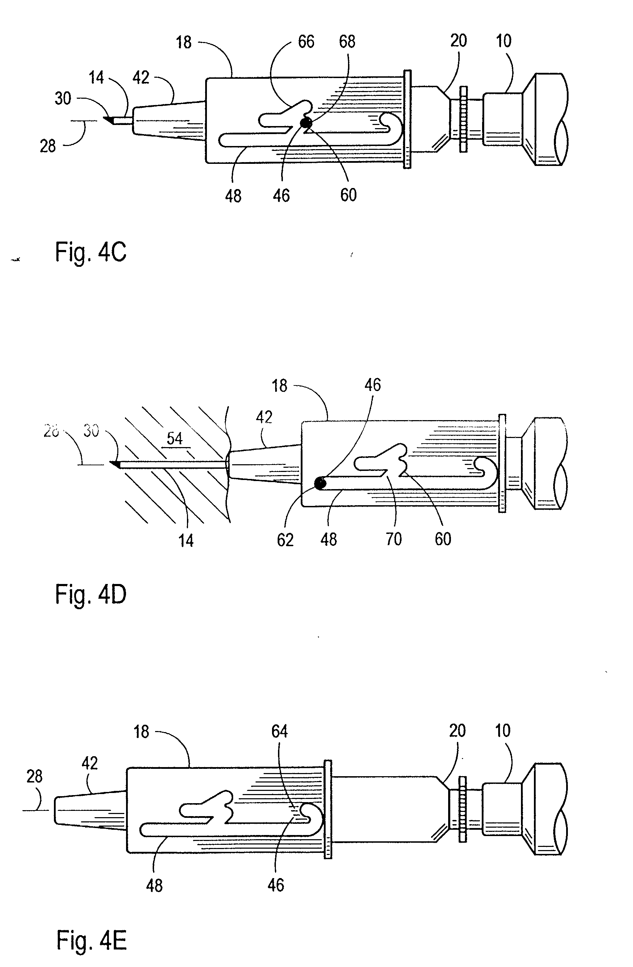

Referring to Fig. 2, it is to be appreciated that movement between the adapter 20 and barrel piece 18 is driven by two opposing forces, an internal force caused by a spring 50 and an external force arising when the nose 42 of the barrel piece 18 is pressed against a surface such as the medicament vial 52 (shown in Fig. 4B) or the patient 54 (shown in Fig. 4D). The branched shape of the slot 48 is designed to regulate the distance the distal end 30 of the needle 14 extends from the nose 42 of the barrel piece 18.

Figs. 4A through 4E show a sequence of steps that include filling the syringe 10 with fluid medicament from a medicament vial 52 and then injecting a patient 54 with the medicament. During this sequence, as shown, the nose 42 of the barrel piece 18 is pressed against the medicament vial 52, then released, pressed against the skin of the patient 54 and then released. Also during this sequence, the barrel piece 18 is forced to move axially, back and forth, relative to remaining portions of the syringe 10. It is to be appreciated, however, that these movements will be limited by the slot 48 and plug 46 assembly. Specifically, five stop points 56, 58, 60, 62 and 64 formed in the slot 48 are provided to stop and hold the plug 46 and thereby regulate axial movement of the barrel piece 18 initially and during the sequence of steps.

Referring now to Fig. 4A, the slot 48 is configured with a stop point 56 to initially hold the plug 46 in a position where the distal end 30 of the needle 14 extends only slightly from the nose 42 of the barrel piece 18. This slight extension allows the user to locate the tip of the needle 14 in the medicament vial 52 (shown in Fig. 4B). Specifically, the stop point 56 prevents the spring 50 from moving the barrel piece 18 in a distal direction relative to the rest of the syringe 10.

Referring now to Fig. 4B, it can be seen that the nose 42 of the barrel piece 18 is to be pressed against the medicament vial 52 during insertion of the distal end 30 of the needle 14 into the medicament vial 52. As the barrel piece 18 moves in the proximal direction in response to the pressing force caused by the medicament vial 52, the plug 46 relocates in the slot 48 toward stop point 58. When the plug 46 reaches the stop point 58, further proximal movement by the barrel piece 18 due to the pressing force is prevented. Preferably, as shown, the slot 48 is designed with the stop point 58 located at a point to extend the distal end 30 of the needle 14 at a distance that will allow the syringe 10 to be easily filled with medicament from the medicament vial 52.

Referring now to Fig. 4C, the syringe 10 is shown after filling (i.e. the needle 14 is removed from the medicament vial 52 (shown in Fig. 4A), releasing the force on the barrel piece 18 due to the medicament vial 52. Without the external force, the spring 50 (shown in Fig. 2) translates the barrel piece 18 distally to re-cover the portion of the needle 14 that was exposed during filling. During the translation, the plug 46 relocates within the slot 48 towards stop point 60. When the plug 46 reaches stop point 60, further distal movement by the barrel piece 18 by the spring 50 is prevented. As shown, stop point 60 is preferably located in the slot 48 at a point where the distal end 30 of the needle 14 is again only slightly extended from the nose 42 of the barrel piece 18. This slight extension allows the user to locate the distal end 30 of the needle 14 at a suitable injection point.

Comparing Figs. 4A and 4C, it can be seen that stop point 56 and stop point 60 result in approximately the exposure of needle 14, yet stop point 56 and stop point 60 are located at different positions along the slot 48. Specifically, as shown, stop points 56, 58 and 60 are azimuthally offset relative to the needle axis 28. The slot 48 is designed in this manner to allow the plug 46 to sequentially relocate from stop point 56 to stop point 58 and then to stop point 60, in order, during the fill sequence. To achieve this sequential movement of the plug 46, the guide ramp 66 of the slot 48 between stop points 56 and 58 is sloped relative to the needle axis 28. Further, as shown, a guide ramp 68 is formed in the slot 48 between stop points 58 and 60 to catch the plug 46 and guide the plug 46 into stop point 60. Thus, it is to be appreciated that as the plug 46 relocates along the slot 48 through from stop points 56 to stop point 58, the barrel piece 18 and adapter 20 rotate relative to each other due to the slope of the guide ramps 66, 68.

Figure 4D shows the syringe 10 after the syringe 10 has been filled and the distal end 30 of the needle 14 has been inserted into the patient 54 for an injection. During insertion, it is to be appreciated that pressure is exerted on the nose 42 of the barrel piece 18 by the skin of the patient 54. This pressure moves the barrel piece 18 in the proximal direction causing the plug 46 to relocate in the slot 48 toward stop point 62. As shown, stop points 60 and 62 are azimuthally offset relative to the needle axis 28. Guide ramp 70 is formed in the slot 48 between stop points 60 and 62 to catch the plug 46 and guide the plug 46 into stop point 62. Thus guide ramp 70 ensures that the plug 46 sequentially relocates from stop point 60 to stop point 62 during penetration of the skin 54 by the needle 14. Once the plug 46 reaches the stop point 62, further proximal movement by the barrel piece 18 due to the skin pressure is prevented. Through proper positioning of stop point 62 on the barrel piece 18, the depth of needle 14 penetration into the patient 54 can be controlled.

Referring now to Fig. 4E, the syringe 10 is shown after the injection, with the syringe 10 removed from the patient. As shown, the pressure on the barrel piece 18 due to contact with the skin of the patient 54 (shown in Fig. 4D) has been released. Without the external force, the spring 50 (shown in Fig. 2) translates the barrel piece 18 distally to fully cover the exposed portion of the needle 14. During the translation, the plug 46 relocates within the slot 48 towards stop point 64. When the plug 46 reaches the stop point 64, further movement by the barrel piece 18 is prevented. For the present invention, the slot 48 is formed with a detent at stop point 64 to hold and lock the plug 46. Thus, after an injection, the barrel piece 18 becomes locked in a position where the needle 14 is fully covered and protected.

While the particular devices and methods as herein shown and disclosed in detail are fully capable of obtaining the objects and providing the advantages herein before stated, it is to be understood that they are merely illustrative of the presently preferred embodiments of the invention and that no limitations are intended to the details of construction or design herein shown other than as described in the appended claims.

## Claims

1. A device for protecting the hollow needle of a syringe after the medicament chamber of the syringe is filled with a medicament and the medicament is subsequently injected into a patient, said device comprising:
an adapter for holding the hollow needle with the hollow needle extending in a distal direction from said adapter to define an axis, said adapter being formed with a means for attaching said adapter to the medicament chamber of the syringe for fluid communication between the medicament chamber and the hollow needle;
a barrel piece disposed on said adapter for axial movement thereon, said barrel piece having a distal end formed with an aperture for receiving the hollow needle therethrough;
a means for biasing said barrel piece along said axis in a distal direction from said adapter; and
a means for interconnecting said adapter with said barrel piece for initially holding said barrel piece in a first position relative to said adapter, in response to said biasing means, and for sequentially directing said barrel piece through a second position, a third position, a fourth position and into a needle-protecting fifth position relative said adapter in response to a selectively applied force against said barrel piece in opposition to said biasing means.

2. A device as recited in claim 1 wherein said biasing means is a spring.

3. A device as recited in claim 1 wherein said interconnecting means comprises:
a plug formed on said adapter and extending therefrom; and
a slot formed in said barrel piece for engagement with said plug.

4. A device as recited in claim 3 wherein said hollow needle defines an axis and said slot is formed having a first slot portion that is substantially linear and substantially parallel to said axis, said first slot portion having a first end for contact with said plug when said barrel piece is in said fourth position, and a second end formed with a lockout for contact with said plug when said barrel piece is in said fifth position.

5. A device as recited in claim 4 wherein said slot is formed with a second slot portion connected to said first slot portion between said first and second end, said second slot portion oriented at an angle to said first slot portion to guide said plug from said third position to said fourth position in response to said selectively applied force.

6. A device as recited in claim 5 wherein said slot is formed with a third slot portion connected to said second slot portion, said third slot portion oriented at an angle to said second slot portion to guide said plug from said second position to said third position upon release of said selectively applied force.

7. A device as recited in claim 6 wherein said slot is formed with a fourth slot portion connected to said third slot portion, said fourth slot portion oriented at an angle to said third slot portion to guide said plug from said first position to said second position in response to said selectively applied force.

8. A syringe for receiving a fluid medicament from a medicament vial, injecting the fluid medicament into a patient and passively protecting the sharp portions of the syringe thereafter, said syringe having a distal end and a proximal end, said syringe comprising:
a syringe body, said syringe body formed with chamber for holding a fluid medicament;
a hollow needle having a tip and defining an axis;
an adapter for mounting said hollow needle on said syringe body in fluid communication with said chamber and with said tip extending distally from said adapter, said adapter further formed with a plug extending therefrom;
a spring positioned in contact with said adapter; and
a barrel piece disposed on said adapter for movement thereon, said barrel piece formed with an aperture for receiving said hollow needle therethrough, said barrel piece positioned against said spring to bias said barrel piece towards said distal end of said syringe, said barrel piece formed with a slot for engagement with said plug of said adapter, wherein said slot is configured to hold said needle tip at a first distance from said barrel piece aperture, and then in sequence, hold said needle tip at a second distance from said barrel piece aperture in response to a first force applied to said barrel piece and directed toward said proximal end of said syringe, hold said needle tip at a third distance from said barrel piece aperture upon release of said first force, hold said needle tip at a fourth distance from said barrel piece aperture in response to a second force applied to said barrel piece and directed toward said proximal end of said syringe and hold said barrel piece over said needle tip to protect said needle tip upon release of said second force.

9. A device as recited in claim 8 further comprising a plunger for engagement with said chamber to expel fluid medicament from said chamber and through said hollow needle.

10. A device as recited in claim 8 further comprising a cap engageable with said barrel piece to cover said hollow needle while said needle tip is at a first distance from said barrel piece aperture.
